# EUROPEAN PATENT APPLICATION

(11) **EP 4 026 482 A1**
(43) Date of publication of application: **13.07.2022**
(21) Application number: 20859769.0
(22) Date of filing: 02.09.2020
(51) Int. Cl.: A61B 5/00, A61B 5/055, G16H 50/20, G06N 20/00

(54) **APPARATUS FOR HIERARCHICALLY DIAGNOSING BRAIN ATROPHY DISEASE ON BASIS OF BRAIN THICKNESS INFORMATION**

(30) Priority: 03.09.2019 KR 20190109012
(71) Applicant: Korea University Research and Business Foundation, Seoul 02841 (KR); Samsung Life Public Welfare Foundation, Seoul 04348 (KR)
(72) Inventor: SEONG, Jun Kyung, Seoul 02799 (KR); KIM, Jeong Hun, Seoul 02635 (KR); SEO, Sang Won, Seoul 06351 (KR)
(74) Representative: Botti, Mario
(86) International application number: PCT/KR2020/011787
(87) International publication number: WO 2021/045505

(57) **Abstract**

Disclosed is a hierarchical diagnosis device of brain atrophy based on brain thickness information, including a brain structure modeling unit to generate a grey matter surface mesh and a white matter surface mesh by mesh modeling of multiple Magnetic Resonance Imaging (MRI) images; a brain thickness extraction unit to acquire brain thickness information by collecting and analyzing a distance between corresponding points of the grey matter surface mesh and the white matter surface mesh; a training data generation unit to generate and store multiple training data including the brain thickness information and diagnosis information when diagnosis model training is requested; a diagnosis model training unit to classify and analyze the brain thickness information into groups of a hierarchical structure according to the diagnosis information to acquire feature information for each group, and generate and train hierarchical classifiers based on the hierarchical structure of the groups and the feature information for each group; and a brain atrophy diagnosis unit to acquire new brain thickness information upon receiving a new input of an MRI image of a subject, and hierarchically identify and notify a type of brain atrophy corresponding to the new thickness information through the hierarchical classifiers.

## Description

### [Technical Field]

The present disclosure relates to a hierarchical diagnosis device of brain atrophy based on brain thickness information for hierarchically diagnosing brain atrophy, in particular, the subtypes of brain atrophy, by acquiring and learning brain thickness information.

### [Background Art]

With aging population, neurodegenerative diseases such as Alzheimer's disease are increasing. Since neurodegenerative diseases are brain related diseases, noninvasive diagnosis methods are necessary.

The noninvasive diagnosis methods of brain related diseases chiefly include diagnosis methods using Magnetic Resonance Imaging (MRI) of the brain, and they are the best way to diagnose the condition of brain tissues. Since there is no influence of artifact by the skull compared to Computed Tomography (CT) of the brain, it is possible to precisely diagnose lesions in the brain stem, cerebellum and temporal lobe, detect cerebral infarction early, and precisely diagnose brain perfusion and the condition of blood vessels in the brain. MRI is an imaging technique that acquires a cross-sectional image of a human body using a magnetic field generated by the magnetism. In MRI, a variety of images may be acquired by controlling the image parameters, and imaging which allows tissues with short relaxation time to become bright is referred to as T1 weighted imaging, and imaging which allows tissues with long T2 relaxation time to become bright is referred to as T2 weighted imaging. Additionally, the most suitable T2 weighted images for molecule images and cell images may be acquired by controlling the image parameters so that tissues with long T2 relaxation time appear bright in the images.

However, noninvasive brain disease detection has low accuracy.

The existing brain disease analysis device using the noninvasive diagnosis method only can accurately determine whether a person has a brain disease, and derives the significance of lesions observed in MRI images in reliance on a doctor's personal experience to determine the type of disorder, and further, predict the prognosis of the disorder.

Accordingly, there is a growing need for not only brain disease diagnosis but also hierarchical diagnosis of the subtypes of brain atrophy (AD, bvFTD, SD, PNFA), Alzheimer's Disease (AD), Frontotemporal Dementia (FTD), Primary Progressive Aphasia (PPA), Nonfluent/agrammatic Variant PPA (nfvPPA) and Semantic Variant PPA (svPPA).

### [Disclosure]

### [Technical Problem]

The present disclosure is designed to solve the above-described problem, and therefore the present disclosure is directed to providing a hierarchical diagnosis device of brain atrophy based on brain thickness information for diagnosing brain atrophy, and further, hierarchically diagnosing the subtypes of brain atrophy by acquiring and learning brain thickness information using Magnetic Resonance Imaging (MRI) images.

The object of the present disclosure is not limited to the above-mentioned object, and other objects not mentioned herein will be clearly understood by those having ordinary skill in the technical field pertaining to the present disclosure from the following description.

### [Technical Solution]

As a means for solving the above-described problem, according to an embodiment of the present disclosure, there is provided a hierarchical diagnosis device of brain atrophy based on brain thickness information, including a brain structure modeling unit to generate a grey matter surface mesh and a white matter surface mesh by mesh modeling of multiple Magnetic Resonance Imaging (MRI) images; a brain thickness extraction unit to acquire brain thickness information by collecting and analyzing a distance between corresponding points of the grey matter surface mesh and the white matter surface mesh; a training data generation unit to generate and store multiple training data including the brain thickness information and diagnosis information when diagnosis model training is requested; a diagnosis model training unit to classify and analyze the brain thickness information into groups of a hierarchical structure according to the diagnosis information to acquire feature information for each group, and generate and train hierarchical classifiers based on the hierarchical structure of the groups and the feature information for each group; and a brain atrophy diagnosis unit to acquire new brain thickness information upon receiving a new input of an MRI image of a subject, and hierarchically identify and notify a type of brain atrophy corresponding to the new thickness information through the hierarchical classifiers.

The diagnosis model training unit generates and trains a first classifier for classifying the brain atrophy as normal cognition or dementia; a second classifier for classifying as Alzheimer's Disease (AD) or Frontotemporal Dementia (FTD) in case of the dementia; a third classifier for classifying as Behavior Variables FTD (bvFTD) or Primary Progressive Aphasia (PPA) in case of the Frontotemporal Dementia (FTD); and a fourth classifier for classifying as Nonfluent/agrammatic Variant PPA (nfvPPA) or Semantic Variant PPA (svPPA) in case of the Primary Progressive Aphasia (PPA).

Alternatively, the diagnosis model training unit generates and trains a first classifier for classifying the brain atrophy as normal cognition or dementia; a second classifier for classifying as Alzheimer's Disease (AD) or Frontotemporal Dementia (FTD) in case of the dementia; a third classifier for classifying as Behavior Variables FTD (bvFTD) or Nonfluent/agrammatic Variant PPA (nfvPPA) in case of the Frontotemporal Dementia (FTD); a fourth classifier for classifying as Behavior Variables FTD (bvFTD) or Semantic Variant PPA (svPPA) in case of the Frontotemporal Dementia (FTD); and a fifth classifier for classifying as Nonfluent/agrammatic Variant PPA (nfvPPA) or Semantic Variant PPA (svPPA) in case of the Frontotemporal Dementia (FTD).

The brain structure modeling unit further includes a function of matching locations of each vertex on a brain surface by re-sampling a grey matter surface and a white matter surface according to a preset reference template.

In addition, the hierarchical diagnosis device of brain atrophy further includes a denoising unit to remove noise included in each thickness information.

Furthermore, the diagnosis model training unit includes a dimensionality reduction unit to reduce a data dimension of the brain thickness information; and a hierarchical classifier training unit to classify the brain thickness information into groups of a hierarchical structure according to the diagnosis information, acquire feature information for each group through linear determinant analysis, and generate and train hierarchical classifiers based on the hierarchical structure of the groups and the feature information for each group.

### [Advantageous Effects]

The present disclosure extracts brain thickness information for classifying the subtypes of brain atrophy from Magnetic Resonance Imaging (MRI) images, and finally hierarchically diagnoses normal cognition (NC) and the subtypes of brain atrophy (AD, bvFTD, nfvPPA, svPPA) using curvelet-based denoising, linear dimensionality reduction and machine learning algorithms for the extracted brain thickness information, thereby identifying the type of brain atrophy more accurately and precisely.

### [Description of Drawings]

FIG. 1 is a diagram showing a hierarchical diagnosis device of brain atrophy based on brain thickness information according to an embodiment of the present disclosure.
FIG. 2 is a diagram showing a hierarchical structure of brain disease groups.
FIG. 3 is a diagram illustrating a brain atrophy diagnosis method according to an embodiment of the present disclosure.
FIG. 4 is a diagram showing an example of diagnostic modeling according to an embodiment of the present disclosure.
FIG. 5 is a diagram showing an example of diagnostic modeling according to another embodiment of the present disclosure.

### [Best Mode]

The following disclosure describes the principle of the present disclosure. Accordingly, although not explicitly described or illustrated herein, those skilled in the art will realize the principle of the present disclosure and invent a variety of devices included in the concept and scope of the present disclosure. In addition, all the conditional terms and embodiments as used herein are, in principle, obviously intended to help understanding of the concept of the present disclosure, and should not be understood as limited to the particular embodiments and aspects described herein.

In addition, it should be understood that the detailed description of not only the principle, perspective and embodiments of the present disclosure but also particular embodiments is intended to include structural and functional equivalents thereto. Furthermore, these equivalents should be understood as including equivalents that are now well known as well as equivalents that will be developed in the future, i.e., all devices invented to perform the same function irrespective of structure.

Accordingly, for example, it should be understood that any block diagrams herein represent conceptual views of illustrative systems embodying the principles of the present disclosure. Likewise, it should be understood that any flow charts, state transition diagrams, pseudo code and the like represent various processes which may be substantially represented in computer-readable medium and so executed by a computer or processor, whether or not such computer or processor is explicitly shown.

The functions of various devices shown in the drawings including the processor or function blocks indicated in a similar concept may be provided by the use of dedicated hardware as well as hardware capable of running software in association with appropriate software. When provided by the processor, the functions may be provided by a single dedicated processor, a single shared processor or a plurality of individual processors, and some of them may be shared.

In addition, the explicit use of the term processor, control or the term presented in a similar concept should not be construed to refer exclusively to hardware capable of running software, and it should be understood that such terms implicitly include, without limitation, digital signal processor (DSP) hardware and read only memory (ROM), random access memory (RAM) and nonvolatile memory for storing software. Other well-known hardware may also be included.

In the appended claims of the present disclosure, any element expressed as a means for performing the function described in the detailed description is intended to encompass any way of performing the function including, for example, a combination of circuit elements that perform the function or software in any form including firmware, microcode or the like, combined with appropriate circuitry for running the software to perform the function. The present disclosure defined by the appended claims should be understood that the functions provided by the various recited means are combined and brought together in the manner which the appended claims call for, and thus any means that can provide the functions is equivalent to those shown herein.

The above-described objects, features and advantages will be apparent through the following detailed description related to the accompanying drawings, and accordingly those having ordinary skill in the technical field pertaining to the present disclosure will easily carry out the technical spirit of the present disclosure. In addition, in describing the present disclosure, when it is determined that a certain detailed description of known technology related to the present disclosure may unnecessarily obscure the subject matter of the present disclosure, the detailed description is omitted.

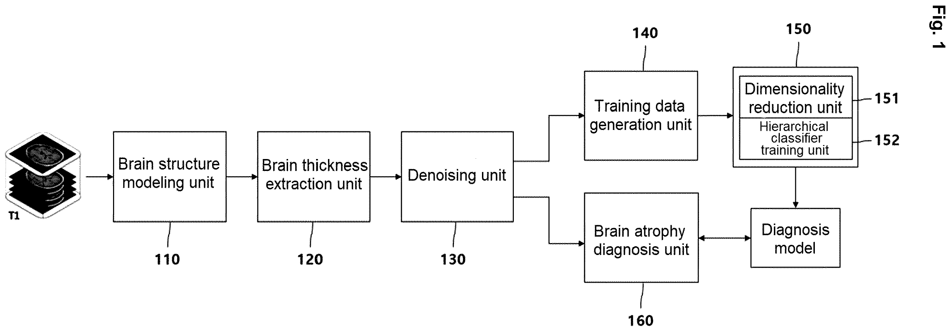

FIG. 1 is a diagram showing a hierarchical diagnosis device of brain atrophy based on brain thickness information according to an embodiment of the present disclosure.

Referring to FIG. 1, the hierarchical diagnosis device 100 of brain atrophy of the present disclosure includes a brain structure modeling unit 110, a brain thickness extraction unit 120, a denoising unit 130, a training data generation unit 140, a diagnosis model training unit 150 and a brain atrophy diagnosis unit 160.

The brain structure modeling unit 110 generates a grey matter surface and a white matter surface having a polygonal mesh data structure by mesh modeling of multiple 3-dimensional volumetric images such as Magnetic Resonance Imaging (MRI) images. In this instance, in particular, the MRI image is preferably a T1 weighted image.

The polygonal mesh is represented as a mesh including faces and vertices, but the number of faces and vertices of the brain surface mesh extracted for each individual may differ, and in this case, comparative analysis between individuals is difficult.

The present disclosure pre-acquires and stores a reference template corresponding to the standard brain structure, and extracts a new vertex of which locations on the brain surface almost match for each vertex ID by re-sampling the grey matter surface and the white matter surface through the reference template, thereby allowing feature comparison between individuals.

The brain thickness extraction unit 120 extracts a pair of vertices (i.e., a pair of corresponding points) matched to each other based on the grey matter surface mesh and the white matter surface mesh acquired through the brain structure modeling unit 110, and acquires brain thickness information by collecting and analyzing the distance between the vertices. That is, a change in grey matter thickness by the brain shrinkage is identified through the brain thickness information.

For reference, the brain surface generated and re-sampled through the brain structure modeling unit 110 has a large amount of noise, and due to the large amount of noise, brain thickness information extracted for each individual also has a large amount of noise. The noise has a locally nonlinear projective property.

The denoising unit 130 of the present disclosure converts the brain thickness information into frequency domain, and removes all high frequency components (i.e., locally nonlinear projective noise components) included in the brain thickness information through a denoising method such as Laplace Beltrami Operator (LBO).

When machine learning is requested, the training data generation unit 140 is selectively activated to acquire brain thickness information and diagnosis information corresponding to each of a plurality of patients and combine them to generate multiple training data.

The diagnosis model training unit 150 includes a dimensionality reduction unit 151 and a hierarchical classifier training unit 152, and generates a diagnosis model for brain atrophy diagnosis by machine learning of the multiple training data.

The dimensionality reduction unit 151 reduces the data dimension of the brain thickness information to lower dimension so that shrinkage can be actually compared through a dimensionality reduction method such as Principal Component Analysis (PCA). That is, as regions of the brain in which the brain shrinks vary depending on the type of brain atrophy, the present disclosure selects each region of the brain in which the brain shrinks as a principal component by the percentage of Table 1 (exemplary percentage) and brings out only the features of the locations, and an example of actual application (an example of application in percentage) may be as shown in Table 2.

**[Table 1]**

| | Percentage of brain thickness information dimension | Percentage of frequency dimension (LB operator) | Percentage of PCA dimension |
|---|---|---|---|
| CN vs Dementia | 100% | about 0.2% | about 0.1% |
| AD vs FTD | 100% | about 0.2% | about 0.1% |
| bvFTD vs PPA | 100% | about 0.2% | about 0.1% |
| nfvPPA vs svPPA | 100% | about 0.2% | about 0.1% |

**[Table 2]**

| | Brain thickness information dimension | Frequency dimension (LB operator) | PCA dimension |
|---|---|---|---|
| CN vs Dementia | 81,924 | 150-170 | 75-85 |
| AD vs FTD | 81,924 | 220-260 | 55-65 |
| bvFTD vs PPA | 81,924 | 250-290 | 90-100 |
| nfvPPA vs svPPA | 81,924 | 200-240 | 75-85 |

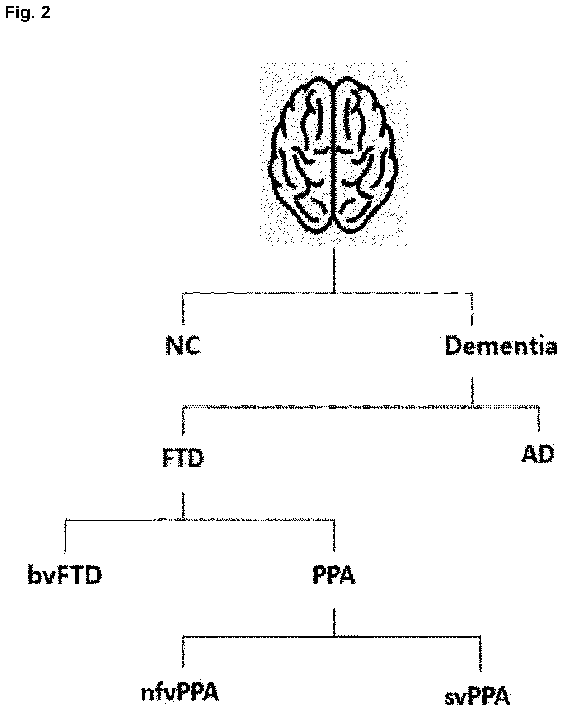

The hierarchical classifier training unit 152 classifies the brain thickness information included in the training data into brain disease groups of hierarchical structure as shown in FIG. 2 according to the diagnosis information, and extracts one-dimensional feature information for each group by calculating and applying a matrix including features with low correlation between groups and features with high correlation within groups through a feature extraction method such as Linear Discriminant Analysis (LDA) and support vector machine (SVM). Additionally, hierarchical classifiers are generated and trained based on the hierarchical structure of the groups and the feature information for each group, and a diagnosis model including the hierarchical classifiers is acquired and stored.

Finally, the brain atrophy diagnosis unit 160 is activated when disease diagnosis is requested. When brain thickness information corresponding to an MRI image of a subject is newly acquired, the brain atrophy diagnosis unit 160 finally identifies and notifies the type of brain atrophy corresponding to the brain thickness information of the subject by hierarchically analyzing the similarity with the brain disease groups through the hierarchical classifiers in the diagnosis model.

As described above, the present disclosure extracts the brain thickness information for classifying the subtypes of brain atrophy from the MRI image, and hierarchically diagnoses brain atrophy as shown in FIG. 2 using curvelet-based denoising, linear dimensionality reduction and machine learning algorithms for the extracted brain thickness information.

That is, the type of brain atrophy of the subject is diagnosed through the procedure of identifying whether the subject has normal cognition (NC) or dementia, and in the case of dementia, identifying whether the subject has Alzheimer's Disease (AD) or Frontotemporal Dementia (FTD), and in the case of FTD, identifying whether the subject has Behavior Variables FTD (bvFTD) or Primary Progressive Aphasia (PPA), and in the case of PPA, identifying whether the subject has Nonfluent/agrammatic Variant PPA (nfvPPA) or Semantic Variant PPA (svPPA). The type of brain atrophy may be variously adjusted as needed. For example, instead of nfvPPA and svPPA, Progressive Nonfluent Aphasia (PNFA) and Semantic Dementia (SD) may be applied.

In addition, the hierarchical diagnosis device of brain atrophy may be implemented as an independent hardware device, but where necessary, may be implemented in the form of a plugin into the existing medical equipment, or a software program which is installed and executed in a medical device.

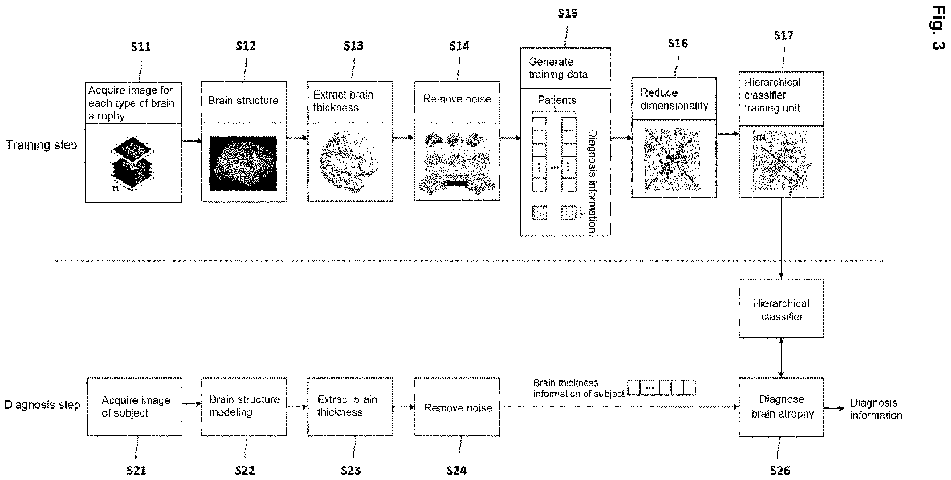

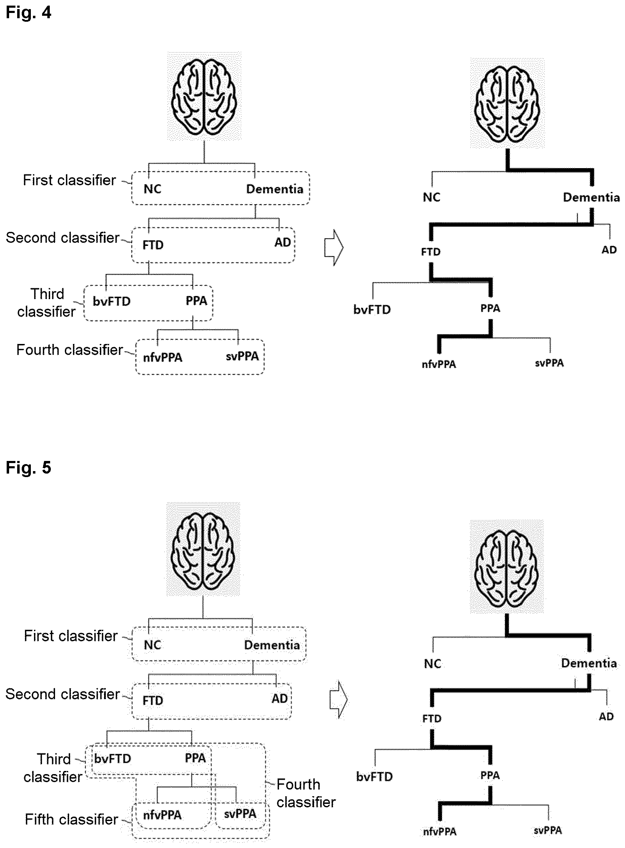

Hereinafter, an operation method of the hierarchical diagnosis device of brain atrophy according to an embodiment of the present disclosure will be described in more detail with reference to FIGS. 3 and 4.

FIG. 3 is a diagram illustrating a brain atrophy diagnosis method according to an embodiment of the present disclosure, and the brain atrophy diagnosis method of the present disclosure largely includes a training step and a diagnosis step.

First, in the training step, MRI images and diagnosis information of a plurality of patients with brain atrophy of NC, AD, bvFTD, nfvPPA and svPPA are acquired by accessing a database server in which the medical images and the diagnosis information of the patients are stored (S11).

Additionally, a grey matter surface mesh and a white matter surface mesh are generated by mesh modeling of each MRI image, and correspondence between the patients is achieved by re-sampling according to a preset reference template (S12).

Additionally, pairs of corresponding points of the re-sampled grey matter surface mesh and white matter surface mesh are all extracted, and brain thickness information of each patient is extracted by collecting and analyzing the distance between the corresponding points (S13), and noise (i.e., harmonics having a locally nonlinear projective property) included in the brain thickness information is removed (S14).

Additionally, patient diagnosis information is added to the brain thickness information acquired through S14 to generate multiple training data (S15).

The generation of the multiple training data is completed through the step S15, and the data dimension of the brain thickness information is reduced through Principal Component Analysis (PCA) (S16).

Additionally, the dimension reduced brain thickness information is classified into hierarchical groups according to the diagnosis information, and feature information for each group is extracted through linear discriminant analysis. Additionally, hierarchical classifiers for hierarchically diagnosing brain atrophy based on the hierarchical structure of the groups and the feature information for each group are generated and trained, and a diagnosis model including the hierarchical classifiers is generated and stored (S17).

When the training step is completed, the diagnosis step may be performed.

When an MRI image of a subject is newly acquired through MRI equipment (S21), brain thickness information of the subject is acquired by the same process as the above-described steps S12 to S15 (S22 to S25).

Then, a group having the highest similarity with the brain thickness information of the subject is hierarchically searched through the hierarchical classifiers of the diagnosis model, and the diagnosis information corresponding to the group having the highest similarity is acquired and outputted as brain atrophy diagnosis information (S26).

FIG. 4 is a diagram showing an example of diagnostic modeling according to an embodiment of the present disclosure, and referring to FIG. 4, the diagnosis model of the present disclosure may include a first classifier for classifying an NC group and a Dementia group, a second classifier for classifying an AD group and an FTD group, a third classifier for classifying a bvFTD group and a PPA group, and a fourth classifier for classifying an nfvPPA group and an svPPA group.

In this circumstance, when brain thickness information of a subject is acquired, brain atrophy may be hierarchically diagnosed based on the brain thickness information using the first to fourth classifiers in a sequential order as follows.

First, (1) one of the NC group and the Dementia group to which the brain thickness information of the subject is more similar is identified through the first classifier.

(2) When the similarity with the NC group is higher, it is determined that the subject has NC, and when the similarity with the Dementia group is higher, similarity with the FTD group and similarity with the AD group are additionally calculated through the second classifier.

(3) When the similarity with the AD group is higher, it is determined that the subject has AD, and when the similarity with the FTD group is higher, similarity with the bvFTD group and similarity with the PPA group are additionally calculated through the third classifier.

(4) When the similarity with the bvFTD group is higher, it is determined that the subject has bvFTD, and when the similarity with the PPA group is higher, similarity with the nfvPPA group and similarity with the svPPA group are additionally calculated through the fourth classifier.

Additionally, (5) when the similarity with the nfvPPA group is higher, it is determined that the subject has nfvPPA, and when the similarity with the svPPA group is higher, it is determined that the subject has svPPA.

FIG. 5 is a diagram showing an example of diagnostic modeling according to another embodiment of the present disclosure, and referring to FIG. 5, the diagnosis model of the present disclosure may include a first classifier for classifying an NC group and a Dementia group, a second classifier for classifying an AD group and an FTD group, a third classifier for classifying a bvFTD group and an nfvPPA group, a fourth classifier for classifying a bvFTD group and an svPPA group, and a fifth classifier for classifying an nfvPPA group and an svPPA group.

In this case, first, (1) one of the NC group and the Dementia group to which brain thickness information of a subject is more similar is identified through the first classifier.

(2) When the similarity with the NC group is higher, it is determined that the subject has NC, and when the similarity with the Dementia group is higher, similarity with the FTD group and similarity with the AD group are additionally calculated through the second classifier.

(3) When the similarity with AD group is higher, it is determined that the subject has AD, and when the similarity with FTD group is higher, similarity with bvFTD group and nfvPPA group, similarity with bvFTD group and svPPA group and similarity with nfvPPA group and svPPA group are respectively calculated using the third classifier, the fourth classifier and the fifth classifier at the same time.

(4) As a result of cross-checking of the third to fifth classifiers, when there is a group having received two votes among the total of three groups (bvFTD, nfvPPA, svPPA), it is determined that the subject has brain atrophy corresponding to the corresponding group, and when each of the total of three groups receives each one vote, brain atrophy is determined on the basis of a group having the greatest distance from the classifier.

As described above, since the present disclosure builds the hierarchical classifiers to hierarchically identify the type of brain atrophy, it is possible to diagnose and notify the type of brain atrophy of the subject more accurately and precisely.

The above-described method according to the present disclosure may be implemented as a program that is executed in a computer and stored in a computer-readable recording medium, and examples of the computer-readable recording medium include ROM, RAM, CD-ROM, magnetic tape, floppy disk and optical data storage devices, as well as those embodied in the form of a carrier wave (for example, transmission via Internet).

The computer-readable recording medium is distributed over computer systems connected via a network, and stores and executes the computer-readable code in a distributed manner. Additionally, functional programs, code and code segments for implementing the method may be easily inferred by programmers in the technical field pertaining to the present disclosure.

Although the preferred embodiments of the present disclosure have been hereinabove illustrated and described, the present disclosure is not limited to the above-described particular embodiments, and a variety of modifications may be made thereto by those having ordinary skill in the technical field pertaining to the present disclosure without departing from the subject matter of the present disclosure claimed in the appended claims, and such modifications should not be individually understood from the technical spirit or scope of the present disclosure.

## Claims

1. A hierarchical diagnosis device of brain atrophy based on brain thickness information, comprising:
a brain structure modeling unit to generate a grey matter surface mesh and a white matter surface mesh by mesh modeling of multiple Magnetic Resonance Imaging (MRI) images;
a brain thickness extraction unit to acquire brain thickness information by collecting and analyzing a distance between corresponding points of the grey matter surface mesh and the white matter surface mesh;
a training data generation unit to generate and store multiple training data including the brain thickness information and diagnosis information when diagnosis model training is requested;
a diagnosis model training unit to classify and analyze the brain thickness information into groups of a hierarchical structure according to the diagnosis information to acquire feature information for each group, and generate and train hierarchical classifiers based on the hierarchical structure of the groups and the feature information for each group; and
a brain atrophy diagnosis unit to acquire new brain thickness information upon receiving a new input of an MRI image of a subject, hierarchically search for a group having highest similarity with the new thickness information through the hierarchical classifiers, and identify and notify a type of brain atrophy based on the group search results.

2. The hierarchical diagnosis device of brain atrophy based on brain thickness information according to claim 1, wherein the diagnosis model training unit generates and trains a first classifier for classifying the brain atrophy as normal cognition or dementia; a second classifier for classifying as Alzheimer's Disease (AD) or Frontotemporal Dementia (FTD) in case of the dementia; a third classifier for classifying as Behavior Variables FTD (bvFTD) or Primary Progressive Aphasia (PPA) in case of the Frontotemporal Dementia (FTD); and a fourth classifier for classifying as Nonfluent/agrammatic Variant PPA (nfvPPA) or Semantic Variant PPA (svPPA) in case of the Primary Progressive Aphasia (PPA).

3. The hierarchical diagnosis device of brain atrophy based on brain thickness information according to claim 1, wherein the diagnosis model training unit generates and trains a first classifier for classifying the brain atrophy as normal cognition or dementia; a second classifier for classifying as Alzheimer's Disease (AD) or Frontotemporal Dementia (FTD) in case of the dementia; a third classifier for classifying as Behavior Variables FTD (bvFTD) or Nonfluent/agrammatic Variant PPA (nfvPPA) in case of the Frontotemporal Dementia (FTD); a fourth classifier for classifying as Behavior Variables FTD (bvFTD) or Semantic Variant PPA (svPPA) in case of the Frontotemporal Dementia (FTD); and a fifth classifier for classifying as Nonfluent/agrammatic Variant PPA (nfvPPA) or Semantic Variant PPA (svPPA) in case of the Frontotemporal Dementia (FTD).

4. The hierarchical diagnosis device of brain atrophy based on brain thickness information according to claim 1, wherein the brain structure modeling unit further includes a function of matching locations of each vertex on a brain surface by re-sampling a grey matter surface and a white matter surface according to a preset reference template.

5. The hierarchical diagnosis device of brain atrophy based on brain thickness information according to claim 1, further comprising:
a denoising unit to remove noise included in each thickness information.

6. The hierarchical diagnosis device of brain atrophy based on brain thickness information according to claim 1, wherein the diagnosis model training unit includes a dimensionality reduction unit to reduce a data dimension of the brain thickness information; and a hierarchical classifier training unit to classify the brain thickness information into groups of a hierarchical structure according to the diagnosis information, acquire feature information for each group through linear determinant analysis, and generate and train hierarchical classifiers based on the hierarchical structure of the groups and the feature information for each group.
